# EUROPEAN PATENT SPECIFICATION

(11) **EP 1 093 810 B1**
(45) Date of publication and mention of the grant of the patent: **13.08.2003**
(21) Application number: 00122652.1
(22) Date of filing: 18.10.2000
(51) Int. Cl.: A61K 9/20, A61K 31/551, A61P 25/20

(54) **Fast-dissolving tablet comprising brotizolam**
Schnelllösliche Tablette, Brotizolam enthaltend
Comprimé contenant brotizolam à dissolution rapide

(30) Priority: 22.10.1999 JP 33833299
(43) Date of publication of application: 25.04.2001
(73) Proprietor: Boehringer Ingelheim International GmbH, 55216 Ingelheim (DE)
(72) Inventor: Wada, Koichi, Takarazuka, Hyogo 666-0874 (JP); Kurosaki, Seisuke, Ikeda, Osaka 563-0023 (JP); Ohki, Toshimitsu, Ikeda, Osaka 563-0043 (JP)
(74) Representative: Kläs, Heinz-Gerd

(56) References cited:
- EP-A- 1 008 343
- GB-A- 1 490 269
- US-A- 5 753 254
- H. SUCKER, P. FUCHS AND P. SPEISER: "Pharmazeutische Technologie", 1991, GEORG THIEME VERLAG, STUTTGART
- Arthur H. Kibbe (Ed.):"Handbook of pharmaceutical excipients" 1986. American Pharmaceutical Association and the Pharmaceutical Press, Washington, pages 53-55

## Description

The present invention relates to a new easy-to-take tablet containing brotizolam rapidly disintegrating in small amounts of water, like for example the saliva of the mouth.

### Background to the invention

Brotizolam is a diazepine with sedative and hypnotic effect. The substance itself and a first manufacturing procedure is disclosed in the German patent DE 2410030. Brotizolam is by far most used as sedative. A major group of customers are elderly people. Brotizolam is available on the market as a tablet that has to be taken orally. The tablet is such that it must be swallowed or chewed by the patient together with water. However, it turned out that the compliance of the elderly for the tablet reduces the more often it has to be taken, especially at night and/or together with water. Among others, this is because elderly people do not like to drink much, especially at night before going to bed and thus prefer taking tablets at night without water.

Another field brotizolam can be used in is as sedative before surgical operations. The advantage of the use of brotizolam in such cases is obvious. However, the fact that it has to be taken together with water reduces the admissibility before operations.

The present invention relates to a new formulation of brotizolam to overcome the aforementioned disadvantages of the present brotizolam tablet. The invention refers to a brotizolam tablet well disintegrating with saliva in the oral cavity and thus being swallowable without the use of water.

While in the state of the art well disintegrating tablets are known, said tablets have several disadvantages. Normally those tablets are very porous and thus not very hard. As a consequence they cannot be broken into two or more pieces, which renders them useless for regiments wherein only one half of a tablet shall be taken at a time. Additionally, these porous tablets tend to be very sensitive to humidity. As a consequence they cannot be stored for some days once the blister is opened.

Another disadvantageous feature of these tablets concerns the manufacturing process. Normally, they are made by compressing wet mass at low pressure followed by drying, for example freeze drying, what is not only rather lavish but also expensive.

### Summary of the invention

Objective of the present invention is to create a tablet containing brotizolam as active ingredient, which easily disintegrates in small amounts of a pharmacologically acceptable aqueous liquid, such as the saliva in the oral cavity while being stable enough for transport and commercial use and which overcomes the disadvantages known in the state of the art.

Another objective of the present invention is to create a tablet containing brotizolam as active ingredient, that easily disintegrates with saliva of the oral cavity while being hard enough to be broken into two stable pieces.

Another object of the present invention is to create a tablet containing brotizolam as active ingredient, that easily disintegrates in saliva in the oral cavity and that resists humidity for several days without taking up moisture or breaking apart if unblistered.

Another object of the present invention is to create a tablet containing brotizolam as active ingredient, which easily disintegrates in saliva in the oral cavity into a well tasting solution or suspension, preferred into a suspension. The well taste of the tablet does not only improve compliance but also minimise the risk that the tablet might be given to a person under misuse intention.

### Description of the invention

The present invention relates to a tablet consisting of
- brotizolam as active agent,
- lactose, crystalline cellulose, corn, maize or potato starch as filling and/or disintegrating agents,
- magnesium stearate, optionally in combination with light anhydrous silicic acid as lubricant and
- a sweetener being selected from the group of aspartame and mono ammonium glycyrrhizinate and
- optionally souring agents selected from the group of mono sodium fumarate and/or citric acid and/or flavouring agents I-menthol, and/or mannitol,
the tablet having a total weight of between 30 and 800 mg, a diameter of 4 to 15 mm and a hardness of 50 N to 100 N to allow disintegrating in up to 2 ml of water or saliva within a maximum of 60 seconds and the tablet being preparable by directly compressing the ingredients at a pressure of 5900 N to 15700 N without using any granulation processes.

The tablet is such that it disintegrates already within up to 60 seconds, more preferred within up to 30 seconds and most preferred within up to 15 seconds. The disintegration process will already take place in a volume of said liquid of up to 2 ml, preferably between 0,5 ml and 2 ml, most preferably already in a volume of 1 ml.

In order to meet the above-mentioned advantages of the tablet, namely being rapidly disintegrating on one hand and being hard enough to be breakable into two pieces, transported and commercialised on the other hand, the tablet must have a certain hardness. It was shown that a hardness of 50 N to 100 N is well suited for the aforementioned purpose. Preferred are tablets with a hardness of 60 N to 90 N, most preferred with a hardness of 65 N to 85 N.

The tablet has a total weight of 30 mg to 800 mg and a diameter of 4 to 15 mm. Preferred are tablets having a total weight of 40 mg to 400 mg and a diameter of 5 mm to 10 mm, preferably 8 mm. Also preferred are tablets with a total weight of 50 mg to 100 mg and a diameter of 5 mm to 7 mm, preferably 6 mm. Most preferred are tablet having a total weight of 150 mg or 170 mg and a diameter of 8 mm and tablets having a total weight of 80 mg or 85 mg and a diameter of 6 mm.

The tablet contains one or more filling and/or disintegrating agent(s). These agents are useful to produce tablets of a certain, size and to support the disintegrating step and are well known in the state of the art. The filling and/or disintegrating agent(s) are selected from the group of lactose, microcrystalline cellulose, starch, preferably corn, maize or potato starch.

The tablet also contains one or more lubricant(s). Lubricants are well known in the state of the art. Among them, the lubricant(s) are selected from the group of light anhydrous silicic acid and/or magnesium stearate. The lubricant may also have a humidity binding effect.

To improve the taste of the tablet it comprises one or more sweetener(s). Theses sweeteners are selected from the group of aspartame and monoammoniumglycyrrhizinate.

Additionally to the sweetener the tablet may optionally comprise one or more souring agent(s), Theses may be selected from the group of mono sodium fumarate and citric acid.

Additionally to the sweetener and souring agent(s), the tablet may optionally comprise one or more flavouring agent(s). The flavouring agents also give the impression of a cooling effect. The flavouring or cooling agent(s) is (are) selected from the group of l-menthol, and/or mannitol.

The tablet is made by using a direct tabletting process, this means that all ingredients are compressed without any granulation processes. The tablet is compressed at a pressure of 5900 N to 15700 N, preferred at a pressure of 7800 N to 14000 N. A tablet with a total weight of 170 mg and a diameter of 8 mm made at a pressure of 9800 N to 12000 N will have a hardness of about 80 N.

The tablet with the aforementioned features and made by the aforementioned process can be used as sedative or in any other indication in that brotizolam may be useful.

The tablet may also be used for rapidly preparing a solution or suspension of brotizolam in an aqueous liquid, within up to 60 seconds, more preferably within a up to 30 seconds, most preferably within up to 15 seconds.

### Examples

### Example 1

| | |
|---|---|
| brotizolam | 0,250 mg |
| lactose | 86,820 mg |
| crystalline cellulose | 30,000 mg |
| corn starch | 30,000 mg |
| aspartame | 0,225 mg |
| monosodium fumarate | 0,750 mg |
| light, anhydrous silicic acid | 1,500 mg |
| l-menthol | 0,007 mg |
| magnesiumstearate | 0,450 mg |
| | |
| total | 150 mg |

### Example 2

| | |
|---|---|
| brotizolam | 0,250 mg |
| lactose | 88,320 mg |
| crystalline cellulose | 30,000 mg |
| corn starch | 30,000 mg |
| aspartame | 0,225 mg |
| monosodium fumarate | 0,750 mg |
| l-menthol | 0,007 mg |
| magnesiumstearate | 0,450 mg |
| | |
| total | 150 mg |

### Example 3

| | |
|---|---|
| brotizolam | 0,250 mg |
| lactose | 89,070 mg |
| crystalline cellulose | 30,000 mg |
| corn starch | 30,000 mg |
| aspartame | 0,225 mg |
| l-menthol | 0,007 mg |
| magnesiumstearate | 0,450 mg |
| | |
| total | 150 mg |

### Example 4

| | |
|---|---|
| brotizolam | 0.250 mg |
| lactose | 99.330 mg |
| microcrystalline cellulose | 34.032 mg |
| corn starch | 34,000 mg |
| aspartame | 0.260 mg |
| mono sodium fumarate | 0.850 mg |
| light anhydrous silicic acid | 0.570 mg |
| l-menthol | 0.008 mg |
| magnesium stearate | 0.700 mg |
| | |
| total | 170 mg |

### Example 5

| | |
|---|---|
| brotizolam | 0.250 mg |
| lactose | 100.180 mg |
| microcrystalline cellulose | 34.032 mg |
| corn starch | 34.000 mg |
| aspartame | 0.260 mg |
| Light anhydrous silicic acid | 0.570 mg |
| l-menthol | 0.008 mg |
| magnesium stearate | 0.700 mg |
| | |
| total | 170 mg |

### Example 6

| | |
|---|---|
| brotizolam | 0.25 mg |
| lactose | 99.338 mg |
| microcrystalline cellulose | 34.032 mg |
| corn starch | 34 mg |
| aspartame | 0.26 mg |
| mono sodium fumarate | 0.85 mg |
| light anhydrous silicic acid | 0.57 mg |
| magnesium stearate | 0.7 mg |
| | |
| total | 170 mg |

### Example 7

| | |
|---|---|
| brotizolam | 0.250 mg |
| lactose | 99.330 mg |
| microcrystalline cellulose | 34.032 mg |
| corn starch | 34.000 mg |
| monoammonium | 0.260 mg |
| glycyrrhizinate | |
| mono sodium fumarate | 0.850 mg |
| light anhydrous silicic acid | 0.570 mg |
| l-menthol | 0.008 mg |
| magnesium stearate | 0.700 mg |
| | |
| total | 170 mg |

### Example 8

| | |
|---|---|
| brotizolam | 0.250 mg |
| lactose | 99.330 mg |
| microcrystalline cellulose | 34.032 mg |
| corn starch | 34.000 mg |
| aspartame | 0.260 mg |
| citric acid | 0.850 mg |
| light anhydrous silicic acid | 0.570 mg |
| l-menthol | 0.008 mg |
| magnesium stearate | 0.700 mg |
| | |
| total | 170 mg |

### Example 9

| | |
|---|---|
| brotizolam | 0.250 mg |
| lactose | 89.410 mg |
| microcrystalline cellulose | 34.032 mg |
| corn starch | 34.00 mg |
| aspartame | 0.260 mg |
| mono sodium fumarate | 0.850 mg |
| light anhydrous silicic acid | 0.570 mg |
| mannitol | 10.000 mg |
| magnesium stearate | 0.700 mg |
| | |
| total | 170 mg |

## Claims

1. Tablet consisting of
- brotizolam as active agent,
- lactose, crystalline cellulose, corn, maize or potato starch as filling and/or disintegrating agents,
- magnesium stearate, optionally in combination with light anhydrous silicic acid as lubricant and
- a sweetener being selected from the group of aspartame and mono ammonium glycyrrhizinate and
- optionally souring agents selected from the group of monosodium fumarate and/or citric acid and/or flavouring agents I-menthol, and/or mannitol,
the tablet having a total weight of between 30 and 800 mg, a diameter of 4 to 15 mm and a hardness of 50 N to 100 N to allow disintegrating in up to 2 ml of water or saliva within a maximum of 60 seconds and the tablet being preparable by directly compressing the ingredients at a pressure of 5900 N to 15700 N without using any granulation processes.

2. Tablet according to claim 1, **characterised in that** it has a hardness of 60 N to 90 N.

3. Tablet according to claim 1, **characterised in that** it has a hardness of 65 N to 85 N.

4. Tablet according to any of claims 1 to 3, **characterised in that** the starch is corn, starch.

5. Tablet according to any of claims 1 to 4, **characterised in that** the tablet comprises light anhydrous silicic acid.

6. Tablet according to any of claims 1 to 5, **characterised in that** the tablet comprises aspartame.

7. Tablet according to any of claims 1 to 6, **characterised in that** the tablet comprises citric acid.

8. Tablet according to any of claims 1 to 7, **characterised in that** it has a total weight of 130 mg to 180 mg and a diameter of 5 mm to 10 mm, preferably 8 mm.

9. Tablet according to any of claims 1 to 7, **characterised in that** it has a total weight of 150 mg or 170 mg and a diameter of 8 mm.

10. Tablet according to any of claims 1 to 7, **characterised in that** it has a total weight of 50 mg to 100 mg and a diameter of 5 mm to 7mm, preferably 6 mm.

11. Method for preparing a tablet according to any one of claims 1 to 10, by directly compressing the ingredients at a pressure of 5900 N to 15700 N without using any granulation processes.

12. Method according of claim 11, **characterised in that** the pressure is 7800 N to 14000 N.

13. Use of a tablet according to any of claims 1 to 10 for the manufacture of a medicament for sedation.

14. Use of a tablet according to any of claims 1 to 10 to be dissolved or suspended in up to 2 ml, preferably 1 ml of water or saliva within up to 60 seconds, preferably within up to 30 seconds.

## Patentansprüche

1. Tablette bestehend aus
- Brotizolam als Wirkstoff
- Lactose, kristalliner Cellulose, Getreide-, Mais- oder Kartoffelstärke als Füllstoff und/oder Zerfallsmittel,
- Magnesiumstearat, gegebenenfalls in Kombination mit leichter wasserfreier Kieselsäure als Gleitmittel und
- einem Süßungsmittel, ausgewählt aus der Gruppe Aspartam und Monoammoniumglycyrrhizinat und
- gegebenenfalls Säuerungsmitteln, ausgewählt aus der Gruppe' Mononatriumfumarat und/oder Citronensäure und/oder Geschmacksverstärkern I-Menthol und/oder Mannit,
wobei die Tablette ein Gesamtgewicht zwischen 30 und 800 mg, einen Durchmesser von 4 bis 15 mm und eine Härte von 50 N bis 100 N, um einen Zerfall in bis zu 2 ml Wasser oder Speichel innerhalb von maximal 60 Sekunden zu ermöglichen, aufweist und die Tablette herstellbar ist durch direktes Pressen der Bestandteile bei einem Druck von 5900 N bis 15700 N ohne Anwendung eines Granulationsverfahrens.

2. Tablette nach Anspruch 1, **dadurch gekennzeichnet, dass** sie eine Härte von 60 N bis 90 N aufweist.

3. Tablette nach Anspruch 1, **dadurch gekennzeichnet, dass** sie eine Härte von 65 N bis 85 N aufweist.

4. Tablette nach irgendeinem der Ansprüche 1 bis 3, **dadurch gekennzeichnet, dass** die Stärke Getreidestärke ist.

5. Tablette nach irgendeinem der Ansprüche 1 bis 4, **dadurch gekennzeichnet, dass** die Tablette leichte wasserfreie Kieselsäure umfasst.

6. Tablette nach irgendeinem der Ansprüche 1 bis 5, **dadurch gekennzeichnet, dass** die Tablette Aspartam umfasst.

7. Tablette nach irgendeinem der Ansprüche 1 bis 6, **dadurch gekennzeichnet, dass** die Tablette Citronensäure umfasst.

8. Tablette nach irgendeinem der Ansprüche 1 bis 7, **dadurch gekennzeichnet, dass** sie ein Gesamtgewicht von 130 mg bis 180 mg und einen Durchmesser von 5 mm bis 10 mm, vorzugsweise 8 mm, aufweist.

9. Tablette nach irgendeinem der Ansprüche 1 bis 7, **dadurch gekennzeichnet, dass** sie ein Gesamtgewicht von 150 mg oder 170 mg und einen Durchmesser von 8 mm aufweist.

10. Tablette nach irgendeinem der Ansprüche 1 bis 7, **dadurch gekennzeichnet, dass** sie ein Gesamtgewicht von 50 mg bis 100 mg und einen Durchmesser von 5 mm bis 7 mm, vorzugsweise 6 mm, aufweist.

11. Verfahren zur Herstellung einer Tablette nach irgendeinem der Ansprüche 1 bis 10 durch direktes Pressen der Bestandteile bei einem Druck von 5900 N bis 15700 N ohne Anwendung eines Granulationsverfahrens.

12. Verfahren nach Anspruch 11, **dadurch gekennzeichnet, dass** der Druck 7800 N bis 14000 N beträgt.

13. Verwendung einer Tablette nach irgendeinem der Ansprüche 1 bis 10 zur Herstellung eines sedativen Arzneimittels.

14. Verwendung einer Tablette nach irgendeinem der Ansprüche 1 bis 10 zur Auflösung oder Suspension in bis zu 2 ml, vorzugsweise 1 ml, Wasser oder Speichel innerhalb von bis zu 60 Sekunden, vorzugsweise innerhalb von bis zu 30 Sekunden.

## Revendications

1. Comprimé consistant en
- brotizolam en tant que principe actif,
- lactose, cellulose cristalline, amidon de maïs (« corn »), amidon de maïs (« maize ») ou fécule de pomme de terre en tant qu'agents de charge et/ou de désagrégation,
- stéarate de magnésium, facultativement en combinaison avec de l'acide silicique anhydre léger en tant que lubrifiant et
- un édulcorant étant choisi dans le groupe de l'aspartame et du glycyrrhizinate de monoammonium et
- facultativement, des agents d'acidification choisis dans le groupe du fumarate monosodique et/ou acide nitrique et/ou des agents de sapidité I-menthol, et/ou mannitol,
le comprimé ayant un poids total entre 30 et 800 mg, un diamètre de 4 à 15 mm et une dureté de 50 N à 100 N pour permettre la désagrégation dans jusqu'à 2 ml d'eau ou de salive dans un laps de temps maximum de 60 secondes et le comprimé pouvant être préparé par compression directe des ingrédients à une pression de 5900 N à 15700 N sans l'utilisation d'aucun procédé de granulation.

2. Comprimé selon la revendication 1, **caractérisé en ce qu'**il présente une dureté de 60 N à 90 N.

3. Comprimé selon la revendication 1, **caractérisé en ce qu'**il présente une dureté de 65 N à 85 N.

4. Comprimé selon l'une quelconque des revendications 1 à 3, **caractérisé en ce que** l'amidon est de l'amidon de maïs (« corn »).

5. Comprimé selon l'une quelconque des revendications 1 à 4, **caractérisé en ce que** le comprimé comprend de l'acide silicique anhydre léger.

6. Comprimé selon l'une quelconque des revendications 1 à 5, **caractérisé en ce que** le comprimé comprend de l'aspartame.

7. Comprimé selon l'une quelconque des revendications 1 à 6, **caractérisé en ce que** le comprimé comprend de l'acide citrique.

8. Comprimé selon l'une quelconque des revendications 1 à 7, **caractérisé en ce qu'**il présente un poids total de 130 mg à 180 mg et un diamètre de 5 mm à 10 mm, de préférence 8 mm.

9. Comprimé selon l'une quelconque des revendications 1 à 7, **caractérisé en ce qu'**il présente un poids total de 150 mg ou 170 mg et un diamètre de 8 mm.

10. Comprimé selon l'une quelconque des revendications 1 à 7, **caractérisé en ce qu'**il présente un poids total de 50 mg à 100 mg et un diamètre de 5 mm à 7 mm, de préférence de 6 mm.

11. Procédé pour la préparation d'un comprimé selon l'une quelconque des revendications 1 à 10, par compression directe des ingrédients à une pression de 5900 N jusqu'à 15700 N sans l'utilisation d'aucun procédé de granulation.

12. Procédé selon la revendication 11, **caractérisé en ce que** la pression est de 7800 N jusqu'à 1400 N.

13. Utilisation d'un comprimé selon l'une quelconque des revendications 1 à 10 pour la fabrication d'un médicament sédatif.

14. Utilisation d'un comprimé selon l'une quelconque des revendications 1 à 10 à dissoudre ou à mettre en suspension dans jusqu'à 2 ml, de préférence 1 ml, d'eau ou de salive dans un laps de temps jusqu'à 60 secondes, de préférence dans un laps de temps jusqu'à 30 secondes.
